# EUROPEAN PATENT APPLICATION

(11) **EP 4 841 251 A1**
(43) Date of publication of application: **18.11.2026**
(21) Application number: 25747668.9
(22) Date of filing: 21.01.2025
(51) Int. Cl.: G01N 27/30, G01N 27/327, G01N 27/416, A61B 5/1473, A61B 5/145, A61B 5/1486

(54) **BIOSENSOR, ISOLATION LAYER THEREOF AND ANALYTE MONITORING APPARATUS**

(30) Priority: 01.02.2024 CN 202410144883
(71) Applicant: SHENZHEN SISENSING CO., LTD., Shenzhen Guangdong 518110 (CN)
(72) Inventor: HAN, Xiongqi, Shenzhen, Guangdong 518110 (CN); FANG, Junfei, Shenzhen, Guangdong 518110 (CN); XIE, Fang, Shenzhen, Guangdong 518110 (CN)
(74) Representative: Vossius & Partner Patentanwälte Rechtsanwälte mbB
(86) International application number: PCT/CN2025/073764
(87) International publication number: WO 2025/162096

(57) **Abstract**

The present disclosure provides a biosensor, an isolation layer thereof, and an analyte monitoring device, the biosensor includes a first electrode provided with a sensing layer, and a polymer membrane covers at least the sensing layer and the first electrode, the polymer membrane includes a protective layer and an isolation layer, the protective layer is closer to the first electrode than the isolation layer, the isolation layer is configured to reduce the effect of a sterilizing gas on the sensing layer when the biosensor is subjected to gas sterilization, the isolation layer is made of a first solution, the first solution includes a first-type polymer and an organic solvent, the first-type polymer is polyurethane. In accordance with the present disclosure, a biosensor resistant to sterilizing gas, an isolation layer thereof, and an analyte monitoring device are provided.

## Description

### BACKGROUND OF INVENTION

The present disclosure relates to the field of new chemical materials, and in particular, to a biosensor, an isolation layer thereof, and an analyte monitoring device.

### Description of Related Art

Biosensors are analytical devices that combine biological materials, biologically derived materials, or biomimetic materials with optical, electrochemical, temperature, piezoelectric, magnetic, or micromechanical physico-chemical sensors or sensing microsystems, and are commonly used to rapidly detect certain specific chemical substances in the human body, such as glucose, ketone bodies, and uric acid.

A biosensor is usually used in combination with an electronic device to form a monitoring system. During detection, a probe of a biosensor containing a sensitive substance (such as an enzyme, an antibody, or a ligand) capable of sensing an analyte is generally placed subcutaneously in the host for detection, and then the detected signal is transmitted to the electronic device for analysis or transmission. Therefore, for the health and safety of the host, it is necessary to sufficiently sterilize and disinfect the probe before use. Common sterilization methods include the gas sterilization method and the irradiation sterilization method. Since electronic devices may be damaged during irradiation sterilization, the gas sterilization method is typically used to sterilize electronic devices. However, the sterilizing gas (such as ethylene oxide) used in the gas sterilization method may affect the sensitive substances on the probe and the performance of the probe itself (such as degradation, denaturation, unwanted chemical reaction, or decomposition of sensitive substances), resulting in a significant decrease in the sensitivity of the biosensor after sterilization by the gas sterilization method, rendering it unable to function normally.

Thus, in existing manufacturing processes, the electronics in the monitoring system are typically separated from the biosensor, and the biosensor is sterilized by the irradiation sterilization method. However, this separate sterilization approach is not conducive to a "one-piece" product design, and the processing technology for separate sterilization is relatively complex. After sterilization, the electronic device and the biosensor must be assembled before use, making the operation relatively complicated.

### SUMMARY OF INVENTION

The present disclosure has been made in view of the above-mentioned state of the art, and its objective is to provide a biosensor resistant to sterilizing gas, an isolation layer thereof, and an analyte monitoring device.

To this end, a first aspect of the present disclosure provides a biosensor for monitoring an analyte, the biosensor comprising a first electrode provided with a sensing layer, and a polymer membrane that covers at least the sensing layer and the first electrode; the polymer membrane includes a protective layer and an isolation layer, the protective layer being closer to the first electrode than the isolation layer, the isolation layer being configured to reduce the effect of a sterilizing gas on the sensing layer when the biosensor being subjected to gas sterilization, the isolation layer is made of a first solution, the first solution including a first-type polymer and an organic solvent, wherein the first-type polymer is polyurethane.

In the first aspect of the present disclosure, the polymer membrane of the biosensor includes a protective layer and an isolation layer. By providing an isolation layer that includes polyurethane, the permeation of sterilizing gas can be limited during gas sterilization of the biosensor, forming a barrier that reduces the influence of the sterilizing gas on the sensing layer, particularly the influence of gaseous ethylene oxide on the active substance in the sensing layer. Thus, the biosensor can be sterilized using a gas sterilization method. Compared to biosensors that do not include an isolation layer, the decrease in sensitivity of the biosensor of the present disclosure before and after sterilization is greatly reduced, and the biosensor can still maintain high sensitivity after sterilization; furthur the isolation layer containing polyurethane has good biocompatibility, which can improve the safety of the biosensor when it is located subcutaneously in the host; in addition, the isolation layer also has a permeation-limiting effect on specific analytes (such as glucose), and can function as a semi-permeable membrane; furthermore, the protective layer separates the isolation layer from the internal structure (the sensing layer and the first electrode), reducing adverse effects of organic solvents in the isolation layer on the sensing layer and the first electrode (e.g., corrosion leading to compromised internal structure integrity and loss of functionality). Accordingly, a biosensor resistant to sterilizing gas can be provided.

In the biosensor according to the first aspect of the present disclosure, optionally, the mass fraction of the first-type polymer in the first solution is from 0.1% to 15%. Thus, the isolation layer can help limit the permeation of the sterilizing gas during gas sterilization.

In the biosensor according to the first aspect of the present disclosure, optionally, the first-type polymer has a soft segment, and the proportion of the soft segment in the first-type polymer is from 5% to 50%. Since the proportion of the soft segment affects the properties of the polyurethane material, in this case, selecting an appropriate proportion of the soft segment enables the polyurethane material to exhibit good mechanical properties, biocompatibility, and the like.

In the biosensor according to the first aspect of the present disclosure, optionally, the first-type polymer has a hydrophilic group, and the proportion of the hydrophilic group in the first-type polymer is from 1% to 30%. The proportion of hydrophilic groups in polyurethane affects the properties of polyurethane materials; in this case, selecting an appropriate proportion of hydrophilic groups enables the polyurethane material to exhibit good hydrophilicity, biocompatibility, and the like.

In the biosensor according to the first aspect of the present disclosure, optionally, the organic solvent is a mixed solvent of any one or more of tetrahydrofuran, dichloromethane, and N,N-dimethylacetamide. Thus, the first-type polymer can be dissolved using the organic solvent.

In the biosensor according to the first aspect of the present disclosure, optionally, the isolation layer has a thickness of from 1 µm to 40 µm. In this case, selecting an appropriate thickness of the isolation layer enables it to effectively function as a barrier, thereby enhancing the biosensor's resistance to sterilizing gas.

In the biosensor according to the first aspect of the present disclosure, optionally, the protective layer includes a second-type polymer, which is a zwitterionic polymer or a copolymer formed from neutral monomers. In this case, the protective layer does not react with the two polymers in the isolation layer, enabling formation of a protective layer that spaces the isolation layer apart from the internal structure (the sensing layer and the first electrode); and the second-type polymer can be dissolved using a low-corrosive or non-corrosive solvent, so that the formed protective layer does not damage the internal structure upon direct contact; in addition, the protective layer imposes minimal restriction on analyte permeation, facilitating analyte passage through the protective layer to reach the sensing layer.

In the biosensor according to the first aspect of the present disclosure, optionally, the protective layer has a thickness of from 1 µm to 20 µm. Thus, the protective layer can effectively separate the isolation layer from the internal structure.

In the biosensor according to the first aspect of the present disclosure, optionally, the polymer membrane further includes a biocompatible layer, the isolation layer being located between the biocompatible layer and the protective layer, wherein the biocompatible layer includes a third-type polymer, which is a hydrophilic group-containing polyurethane or a cross-linked water-soluble polyurethane. In this case, the third-type polymer is also a polyurethane, which, on the one hand, can further enhance the biocompatibility of the biosensor; and, on the other hand, it can also partially block the entry of sterilizing gas during biosensor sterilization, thereby further improving the protective effect of the polymer membrane on the biosensor.

In the biosensor according to the first aspect of the present disclosure, optionally, when the third-type polymer is the hydrophilic group-containing polyurethane, the proportion of the hydrophilic group in the third-type polymer is from 20% to 50%. Thus, the biocompatible layer containing the third-type polymer can exhibit good biocompatibility, thereby further enhancing the biocompatibility of the biosensor.

In the biosensor according to the first aspect of the present disclosure, optionally, the biocompatible layer has a thickness of from 1 µm to 30 µm, thereby helping improve the biocompatibility of the biosensor.

In the biosensor according to the first aspect of the present disclosure, optionally, the sensing layer includes one or more of glucose oxidase, lactate oxidase, and hydroxybutyrate dehydrogenase. Thus, an appropriate sensitive substance can be selected according to the type of analyte.

In the biosensor according to the first aspect of the present disclosure, optionally, the biosensor further includes a second electrode different from the first electrode, and the polymer membrane also covers the second electrode. Thus, the polymer membrane can protect the second electrode.

A second aspect of the present disclosure provides an isolation layer for a biosensor, wherein the biosensor includes a first electrode provided with a sensing layer, the isolation layer at least covering the sensing layer and the first electrode, the isolation layer being configured to reduce the influence of the sterilizing gas on the sensing layer during gas sterilization of the biosensor, the isolation layer being made from a first solution, the first solution including a first-type polymer and an organic solvent, the first-type polymer being polyurethane.

In the second aspect of the present disclosure, the isolation layer including polyurethane limits the permeation of sterilizing gas during gas sterilization of the biosensor, forming a barrier that reduces the impact of sterilizing gas on the sensing layer, particularly the effect of gaseous ethylene oxide on the active substances in the sensing layer, enabling the biosensor to be sterilized using a gas sterilization method; compared to biosensors without an isolation layer, the decrease in sensitivity before and after sterilization is significantly reduced, and high sensitivity is maintained after sterilization; and the isolation layer containing polyurethane has good biocompatibility, which can improve the safety of the biosensor when it is located under subcutaneously in the host; in addition, the isolation layer exhibits a permeation-limiting effect on specific analytes (e.g., glucose), functioning as a semi-permeable membrane. Thus, an isolation layer with shielding effectiveness against sterilizing gas is provided, and when applied to a biosensor, it enables the biosensor to resist sterilizing gas.

A third aspect of the present disclosure provides an analyte monitoring device including an electronic component and the biosensor according to the first aspect of the present disclosure, the electronic component being electrically connected to the biosensor. In the third aspect of the present disclosure, since the biosensor is resistant to sterilizing gas, the biosensor and the electronic component can be assembled together and subjected to gas sterilization, eliminating the need for separate radiation sterilization of the biosensor; the analyte monitoring device can therefore be fully assembled prior to shipment, and the user does not need to assemble the electronic component with the biosensor at the time of use, thereby facilitating ease of use.

In accordance with the present disclosure, a biosensor resistant to sterilizing gas, an isolation layer thereof, and an analyte monitoring device are provided.

### BRIEF DESCRIPTION OF THE DRAWINGS

FIG. 1 is a diagram showing an application scenario of the biosensor according to an example of the present disclosure.
FIG. 2 is a schematic diagram showing the biosensor according to an example of the present disclosure.
FIG. 3 is a schematic diagram showing the polymer membrane according to an example of the present disclosure.
FIG. 4 is a schematic diagram showing the polymer membrane covering an implantable portion according to an example of the present disclosure.
FIG. 5 is a topographical diagram showing the appearance of the biosensor of Example 1 of the present disclosure after sterilization.
FIG. 6 is a topographical diagram showing the appearance of the biosensor of Comparative example 1 of the present disclosure after sterilization.

### DESCRIPTION OF REFERENCE NUMERALS:

100...analyte monitoring device, 1...sensor, 11...implantable portion, 12...connection portion, 13...first electrode, 14...sensing layer, 2...electronic component, 3...polymer membrane, 31...isolation layer, 32...protective layer, 33...biocompatible layer.

### DETAILED DESCRIPTION

Hereinafter, preferred embodiments of the present disclosure will be described in detail with reference to the accompanying drawings. In the following description, the same reference numerals are assigned to the same components, and the repeated description thereof will be omitted. In addition, the drawings are only schematic, and the proportions of the dimensions of the parts relative to each other, the shapes of the parts, etc., may be different from those in practice.

It should be noted that the terms "comprise" and "have", and any variations thereof, for example, a process, method, system, article, or apparatus that includes or has a list of steps or elements in this disclosure are not necessarily limited to those steps or elements expressly listed, but may include or have other steps or elements not expressly listed or inherent to such process, method, article, or apparatus.

A first aspect of the present disclosure relates to a biosensor, which is a biosensor for monitoring an analyte. The biosensors of the present disclosure are resistant to sterilizing gases and can be sterilized using gas sterilization. The biosensors of the present disclosure may be referred to simply as sensors, and may also be referred to as monitoring probes, sensing probes, or electrode probes, and the like.

In the present disclosure, the analyte may be one or more of glucose, acetylcholine, amylase, bilirubin, cholesterol, chorionic gonadotropin, creatine kinase, creatine, creatinine, DNA, fructosamine, glutamine, hormones, ketone body, lactate, oxygen, peroxide, prostate-specific antigen, prothrombin, RNA, thyroid-stimulating hormone, and troponin. By way of example only, the analyte may also be other substances not shown.

A second aspect of the present disclosure is directed to an isolation layer of a biosensor by which the permeation of a sterilizing gas can be limited when the biosensor is subjected to gas sterilization, forming an isolation barrier to reduce the effect of the sterilizing gas on the sensing layer of the biosensor. The isolation layer of the biosensors of the present disclosure may also be referred to as an isolation membrane, a spacer layer, or a sterilization layer, etc.

A third aspect of the present disclosure is directed to an analyte monitoring device, which may include an electronic component and a biosensor. In the analyte monitoring device of the present disclosure, the biosensor is resistant to the sterilizing gas, enabling the biosensor to be assembled with electronic components for gas sterilization without radiation sterilization of the biosensor alone. The analyte monitoring devices of the present disclosure may also be referred to as continuous monitoring devices or monitoring systems, and the like.

Hereinafter, a biosensor (hereinafter may be simply referred to as a sensor) according to the present disclosure will be described with reference to the accompanying drawings, in which an analyte is glucose, for example.

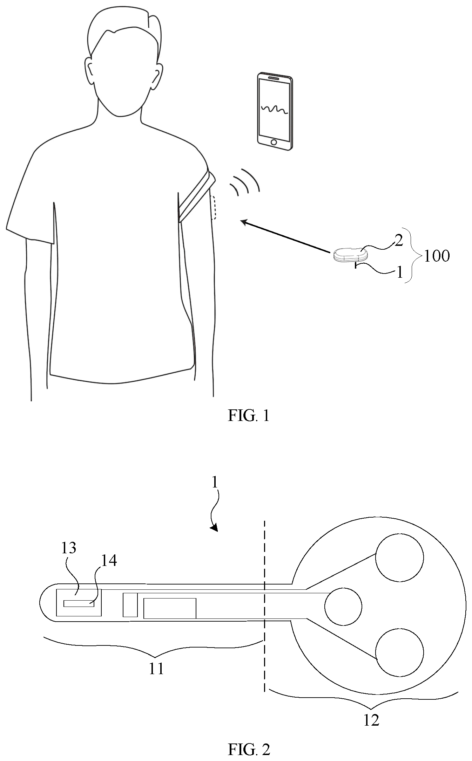

FIG. 1 is a diagram showing an application scenario of sensor 1 according to an example of the present disclosure. FIG. 2 is a schematic diagram showing sensor 1 according to an example of the present disclosure.

In some examples, sensor 1 may be used to monitor an analyte. In some examples, the sensor 1 may be used in conjunction with an electronic component 2. In particular, referring to FIG. 1, a sensor 1 may be combined with an electronic component 2 to form an analyte monitoring device 100 such that the analyte monitoring device 100 is located on a body surface of a host, and such that the sensor 1 is located partially subcutaneously in the host, such that the analyte may be monitored by the sensor 1. It should be noted that although sensor 1 is illustratively described herein as being used in conjunction with electronic component 2 to monitor an analyte in a host, sensor 1 may be placed in a different environment to be tested to detect the analyte, and it need not necessarily be combined with electronic component 2 to form an analyte monitoring device 100 for use, for example, sensor 1 may be electrically connected to an external test circuit for monitoring.

In some examples, the sensor 1 may include an implantable portion 11 (see FIG. 2). The implantable portion 11 may be implanted subcutaneously in the host, and in use, the implantable portion 11 may be located subcutaneously in the host in contact with the analyte. Thus, the analyte in the host can be monitored by the sensor 1. In some examples, the sensor 1 may include a connection portion 12 (see FIG. 2). The implantable portion 11 may be electrically connected to the electronic component 2 via the connection portion 12. In use, the implantable portion 11 may be located subcutaneously, and the connection portion 12 may be located extracorporeally; therefore, the connection portion 12 may also be referred to as an extracorporeal portion.

In some examples, the sensor 1 may include a first electrode 13 (see FIG. 2). A sensing layer 14 may be provided on the first electrode 13. The sensor 1 may react with an analyte via the sensing layer 14 on the first electrode 13 to generate an electrical current signal, which may be analytically processed to obtain an analyte concentration signal. That is, the first electrode 13 may also be referred to as a working electrode.

In some examples, the sensor 1 may include a second electrode. Here, the second electrode may be the same as or different from the first electrode 13. For example, the second electrode may be a reference electrode or a counter electrode. As another example, the second electrode may be a second working electrode, and the analyte detected by the second working electrode may be the same as or different from that detected by the first electrode 13. In some examples, the sensor 1 may include a third electrode. The third electrode may be a reference electrode or a counter electrode and may be different from the second electrode. Thus, a sensor 1 having a three-electrode system can be formed to monitor an analyte.

In some examples, the sensing layer 14 may include a sensitive substance that may promote an analyte reaction. For example, the sensing layer 14 may include one or more of glucose oxidase, lactate oxidase, and hydroxybutyrate dehydrogenase. Thus, a suitable sensitive substance can be selected according to the type of analyte. In some examples, the glucose enzyme may be glucose oxidase or glucose dehydrogenase.

In some examples, the sensor 1 may include a polymer membrane 3 (illustrated later). The polymer membrane 3 can control the diffusion of the analyte. That is, the polymer membrane 3 may be permselective for the analyte. In some examples, the polymer membrane 3 may cover at least the sensing layer 14. Thus, the amount of analyte permeation, i.e., the amount of analyte entering the sensing layer 14, can be controlled by the polymer membrane 3, which can help bring the magnitude of the current signal generated by the analyte in contact with the sensing layer 14 within the linear response range of the sensor 1.

In some examples, the polymer membrane 3 may have biocompatibility. In some examples, the polymer membrane 3 may cover the implantable portion 11 of the sensor 1 (illustrated later). Thus, sensor 1 has biocompatibility.

In some examples, the polymer membrane 3 may limit the permeation of the sterilizing gas when the sensor 1 is subjected to gas sterilization. For example, the polymer membrane 3 may limit the permeation of ethylene oxide (which may be simply referred to as EO). Thus, a sensor 1 resistant to the sterilizing gas can be provided. That is, the sensor 1 of the present disclosure can be sterilized using gas sterilization, and the decrease in sensitivity of the sensor 1 before and after sterilization is greatly reduced compared to a sensor 1 without the polymer membrane 3, and high sensitivity can be maintained after sterilization. In some examples, the polymer membrane 3 may cover at least the sensing layer 14 and the first electrode 13. Thus, corrosion of the internal structure (sensing layer 14 and first electrode 13) by the sterilizing gas during sterilization can be reduced, thereby protecting the internal structure.

In some examples, sensor 1 of the present disclosure may be sterilized using ethylene oxide. The polymer membrane 3 of the present disclosure is subsequently described with reference to ethylene oxide as the sterilizing gas.

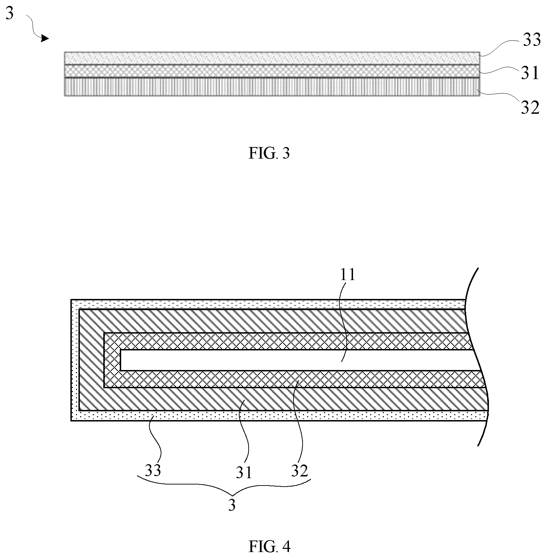

FIG. 3 is a schematic diagram showing the polymer membrane 3 according to an example of the present disclosure.

In some examples, the polymer membrane 3 may include an isolation layer 31 (see FIG. 3). The isolation layer 31 may be configured to reduce the effect of the sterilizing gas on the sensing layer 14 when the sensor 1 is subjected to gas sterilization. In some examples, the isolation layer 31 may be made from a first solution. For example, the first solution may be coated onto the implantable portion 11 to form the isolation layer 31. In some examples, the first solution may be applied to the target location by spray coating, spin coating, dip coating, or the like, to form the isolation layer 31. Here, the target location refers to the place where formation of the isolation layer 31 is desired.

In some examples, the first solution may include a first-type polymer. The first-type polymer may limit the permeation of sterilizing gas (e.g., ethylene oxide). Thus, the isolation layer 31 formed from the first solution including the first-type polymer is capable of limiting the permeation of the sterilizing gas.

In some examples, the first-type polymer may be polyurethane. Polyurethane is a material with good physical and mechanical properties, blood compatibility, and biocompatibility, and ethylene oxide typically does not cause structural changes or degradation of polyurethane when in contact with it at room temperature and atmospheric pressure. In this case, by providing an isolation layer 31 including polyurethane, the permeation of sterilizing gas during gas sterilization of the sensor 1 can be limited, forming a barrier that reduces the impact of sterilizing gas on the sensing layer 14, particularly the effect of gaseous ethylene oxide on active substances (e.g., sensitive substances) in the sensing layer 14; furthermore, the isolation layer 31 containing polyurethane exhibits good biocompatibility, which can improve the safety of the sensor 1 when implanted subcutaneously in a host; in addition, the isolation layer 31 has a permeation-limiting effect on specific analytes (e.g., glucose), enabling the polymer membrane 3 to function as a semi-permeable membrane. In some examples, the first-type polymer may have urethane groups.

In some examples, the first-type polymer may have soft segments, and the proportion of soft segments in the first-type polymer may range from 5% to 50%. In this case, the properties of the first-type polymer can be tuned by adjusting the proportion of soft segments. In some examples, preferably, the proportion of soft segments is from 10% to 25%. In some examples, when the first-type polymer is polyurethane, the polyurethane may have soft segments. Since the proportion of the soft segment affects the properties of the polyurethane material, in this case, selecting an appropriate proportion of the soft segment enables the polyurethane material to exhibit good mechanical properties, biocompatibility, and the like. In some examples, the elasticity, modulus, softness, and water absorption of polyurethane can be adjusted by varying the proportion of soft segments in the polyurethane, thereby regulating the permeability of the isolation layer 31 to the analyte and the biocompatibility of the isolation layer 31 itself. In some examples, the soft segment may include a component containing epoxy functional groups. For example, the soft segment may include polyethylene oxide (PEO).

In some examples, the polyurethane may be a block copolymer composed of soft segments and hard segments. In some examples, in polyurethane, the soft segments may be composed of hydroxyl-containing molecules such as polyester polyols, polyether polyols, polycaprolactones, polylactic-co-glycolic acid copolymers, and the like, with varying molecular weights. In some examples, in polyurethane, the hard segments may be composed of compounds such as diisocyanates and low molecular weight short-chain extenders. In some examples, the diisocyanate may be an aliphatic isocyanate. For example, the diisocyanate may be 1,6-hexamethylene diisocyanate (HDI), 4,4'-dicyclohexylmethane diisocyanate (HMDI), or isophorone diisocyanate (IPDI). In some examples, the short-chain extender may be 1,4-butanediol.

In some examples, the polyurethane may be polycarbonate polyurethane, polyether-based polyurethane, and/or polyester-based polyurethane. In some examples, the polyurethane may include one or more of aliphatic polyether-based polyurethane, aliphatic polyether-based polyurethane urea, cycloaliphatic polyether-based polyurethane, cycloaliphatic polyether-based polyurethane urea, aromatic polyether-based polyurethane, aromatic polyether-based polyurethane urea, aliphatic polyester-based polyurethane, aliphatic polyester-based polyurethane urea, cycloaliphatic polyester-based polyurethane, cycloaliphatic polyester-based polyurethane urea, aromatic polyester-based polyurethane, and aromatic polyester-based polyurethane urea.

In some examples, the first-type polymer may be a zwitterionic polyurethane. In this case, this type of polyurethane has the softness and scalability of polyether chains while having the characteristics of zwitterionic groups, and zwitterionic groups with different characteristics can be introduced as needed to adjust the properties of the isolation layer 31. In some examples, the zwitterionic groups in the zwitterionic polyurethane can include amine groups, carboxyl groups, and/or sulfonic acid groups, among others. In this case, the hydrophilicity and biocompatibility of the isolation layer 31 can be improved by introducing a specific zwitterionic group.

In some examples, the first-type polymer may have hydrophilic groups. Thus, the first-type polymer can be made to have a certain hydrophilicity, which is advantageous for improving the biocompatibility of the isolation layer 31; and ethylene oxide can react with some types of hydrophilic groups, namely, the first-type polymer containing hydrophilic groups capable of reacting with ethylene oxide can adsorb ethylene oxide to a certain extent, thereby further improving the isolation effect of the isolation layer 31 on ethylene oxide. In some examples, the hydrophilic groups may include hydroxyl groups, carboxyl groups, and/or ether groups, among others.

In some examples, the proportion of hydrophilic groups in the first-type polymer may be from 1% to 30%. For example, in the first-type polymer, the proportion of hydrophilic groups may be 1%, 2%, 5%, 6%, 8%, 10%, 12%, 13%, 15%, 16%, 17%, 18%, 20%, 22%, 25%, 26%, 27%, 28%, 29%, or 30%. The proportion of hydrophilic groups in the first-type polymer affects the properties of the first-type polymer, and in this case, the properties of the first-type polymer can be adjusted by adjusting the proportion of hydrophilic groups, thereby adjusting the properties of the isolation layer 31.

In some examples, where the first-type polymer is polyurethane, the proportion of hydrophilic groups may be from 1% to 30%. In this case, by selecting a suitable proportion of hydrophilic groups, the polyurethane material can have good hydrophilicity and biocompatibility, etc.; furthermore, the polyurethane containing hydrophilic groups capable of reacting with ethylene oxide can absorb part of the ethylene oxide to introduce epoxy groups during sterilization, thereby further improving the isolation effect of the isolation layer 31 on the sterilizing gas. In some examples, preferably, the proportion of hydrophilic groups in the first-type polymer may be from 10% to 25%. In some examples, the permeability coefficient of the isolation layer 31 to the analyte may be adjusted by adjusting the hydrophilic groups in the polyurethane. For example, the dispersibility of the polyurethane in the liquid phase may be increased by increasing the proportion of hydrophilic groups in the polyurethane, thereby contributing to an increase in the permeability of the isolation layer 31 to the analyte. Conversely, the permeability of the isolation layer 31 to the analyte can be reduced by reducing the proportion of hydrophilic groups in the polyurethane. In some examples, the hydrophilicity of the polyurethane may be increased by introducing a block containing a hydrophilic group.

In some examples, the first solution may include an organic solvent. The first-type polymer may be dissolved in an organic solvent. Thus, it is possible to facilitate the formation of the first solution and subsequently the formation of the isolation layer 31. In some examples, the organic solvent may be a mixed solvent of any one or more of tetrahydrofuran, dichloromethane, and N,N-dimethylacetamide. Thus, the first-type polymer can be dissolved using an organic solvent.

In some examples, the mass fraction of the first-type polymer in the first solution may be from 0.1% to 15%. For example, the mass fraction of the first-type polymer in the first solution may be 0.1%, 0.5%, 1%, 2%, 3%, 4%, 5%, 6%, 7%, 8%, 9%, 10%, 11%, 12%, 13%, 14%, or 15%. Thus, the isolation layer 31 can help restrict the permeation of the sterilizing gas during gas sterilization. In some examples, preferably, the mass fraction of the first-type polymer in the first solution may be from 0.1% to 5%. In this case, the isolation layer 31 is capable of limiting the permeation of the sterilizing gas while also having an appropriate analyte permeation coefficient.

In some examples, the isolation layer 31 may have a thickness of 1 µm to 40 µm. For example, the isolation layer 31 may have a thickness of 1 µm, 5 µm, 8 µm, 10 µm, 12 µm, 15 µm, 18 µm, 20 µm, 22 µm, 25 µm, 28 µm, 30 µm, 32 µm, 35 µm, 38 µm, or 40 µm. In this case, selecting an isolation layer 31 with a suitable thickness can effectively act as an isolation barrier, thereby improving the resistance of sensor 1 to the sterilizing gas; in addition, it is also possible to provide the isolation layer 31 with a suitable analyte permeation coefficient. In some examples, preferably, the isolation layer 31 may have a thickness of 10 µm to 30 µm. In this case, it is facilitated to balance the isolation effect of the isolation layer 31 on the sterilizing gas and the analyte permeation coefficient.

In some examples, the polymer membrane 3 may include a protective layer 32 (see FIG. 3). In some examples, the protective layer 32 may be used to separate the sensing layer 14 from the isolation layer 31. That is, the protective layer 32 may be closer to the first electrode 13 than the isolation layer 31. Thus, the adverse effects of the organic solvent in the isolation layer 31 on the sensing layer 14 and the first electrode 13 (e.g., corrosion resulting in deterioration of the integrity of the internal structure and loss of functionality) can be mitigated.

In some examples, the protective layer 32 may be made from a second solution. In some examples, the second solution may be coated onto the implantable portion 11 to form the protective layer 32. In some examples, the protective layer 32 may be formed by applying the second solution to the target site via spraying, spin coating, dip coating, or the like. In some examples, the second solution may include a second-type polymer. In other words, the protective layer 32 may include a second-type polymer. The second-type polymer may be non-reactive with the first-type polymer. Thus, a protective layer 32 can be formed that spaces the isolation layer 31 apart from the internal structure (the sensing layer 14 and the first electrode 13).

In some examples, the second-type polymer may be a zwitterionic polymer or a copolymer formed from neutral monomers. In this case, the two polymers in the isolation layer 31 do not react with the protective layer 32; and a low-corrosive or non-corrosive solvent can be used to dissolve the second-type polymer, so that the formed protective layer 32 does not damage the internal structure (the sensing layer 14 and the first electrode 13) upon direct contact; furthermore, the formed protective layer 32 imposes minimal restriction on analyte permeation, facilitating analyte passage through the protective layer 32 to contact the sensing layer 14; in addition, the second-type polymer is also insoluble in the organic solvent of the first solvent, which reduces the possibility of the delamination of membrane layers and inhibits interaction between the two membrane layers, thereby improving the overall stability of the polymer membrane 3.

In some examples, the second-type polymer may be chitosan or carboxymethacrylate.

In some examples, the second-type polymer may be selected from one or more of the following: polyethylene glycol-block-polystyrene, polyacrylic acid-block-polystyrene, polyacrylic acid-co-polystyrene, polyacrylamide-block-polystyrene, polyacrylamide-co-polystyrene, poly(2-vinylpyridine)-block-polystyrene, poly(4-vinylpyridine)-co-polystyrene, poly(4-vinylpyridine)-co-polyvinylpyrrolidone, poly(2-vinylpyridine)-co-polystyrene, poly(4-vinylpyridine)-block-polystyrene, poly(4-vinylpyridine)-co-polyacrylamide, poly(ethoxyethyl acrylate)-co-poly(hydroxyethyl acrylate), and poly(ethoxypropyl acrylate)-co-poly(vinyl alcohol).

In some examples, the solvent of the second solution may be a lower alcohol solution, an inorganic salt buffer solution, or a mixed solution of a lower alcohol and an inorganic salt buffer solution. Thus, the second polymer can be dissolved, facilitating the subsequent application of the second solution. In some examples, the solvent of the second solution may be methanol, ethanol, or a mixed solution of ethanol and phosphate buffer.

In some examples, the protective layer 32 may have a thickness of 1 µm to 20 µm. For example, the protective layer 32 may have a thickness of 1 µm, 2 µm, 3 µm, 4 µm, 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, or 20 µm. Thus, the isolation layer 31 can be effectively spaced apart from the internal structures (the sensing layer 14 and the first electrode 13) by the protective layer 32. In some examples, preferably, the protective layer 32 has a thickness of 8 µm to 15 µm. In some examples, preferably, the protective layer 32 has a thickness of not less than 10 µm.

In some examples, the thickness ratio of the protective layer 32 to the isolation layer 31 may be 1:1 to 1:10. For example, the thickness ratio of the protective layer 32 to the isolation layer 31 may be 1:1, 1:1.5, 1:2, 1:2.5, 1:3, 1:3.5, 1:4, 1:4.5, 1:5, 1:5.5, 1:6, 1:6.5, 1:7, 1:7.5, 1:8, 1:8.5, 1:9, 1:9.5, or 1:10. Thus, it can be advantageous to space the isolation layer 31 from the internal structures (the sensing layer 14 and the first electrode 13) by the protective layer 32. In some examples, preferably, the thickness ratio of the protective layer 32 to the isolation layer 31 is from 1:1 to 1:5.

In some examples, the polymer membrane 3 may include a biocompatible layer 33 (see FIG. 3). The biocompatible layer 33 may have biocompatibility and serve as the outermost layer of the polymer membrane 3 (i.e., the membrane layer farthest from the first electrode 13). In this case, it can be advantageous to improve the biocompatibility of sensor 1.

In some examples, the isolation layer 31 may be located between the biocompatible layer 33 and the protective layer 32. It should be noted that in the present disclosure, the isolation layer 31 can be made to have good biocompatibility by adjusting the isolation layer 31, and the additional biocompatible layer 33 is added for the purpose of further improving the biocompatibility of the sensor 1 and improving reliability, and the biocompatible layer 33 does not necessarily need to be provided.

In some examples, the biocompatible layer 33 may be made from a third solution. In some examples, a third solution may be coated on the implantable portion 11 to form the biocompatible layer 33. In some examples, the biocompatible layer 33 may be formed by applying the third solution to the target site via spray coating, spin coating, dip coating, or the like. In some examples, the third solution may include a third-type polymer. In other words, the biocompatible layer 33 may include a third-type polymer. Wherein, the third-type polymer may be a polymer having biocompatibility. Thus, it is possible to form the biocompatible layer 33 having biocompatibility, thereby contributing to the improvement of the biocompatibility of the sensor 1.

In some examples, the third-type polymer may be polyurethane. In this case, the third-type polymer is also polyurethane, which, on the one hand, makes it possible to further improve the biocompatibility of the sensor 1; on the other hand, it can also partially block the entry of sterilizing gas during the sterilization process of the sensor 1, further improving the protective effect of the polymer membrane 3 on the sensor 1.

In some examples, the third-type polymer may be a hydrophilic group-containing polyurethane. In this case, the biocompatible layer 33 including the third-type polymer can have good biocompatibility; in addition, the hydrophilic group-containing polyurethane having a hydrophilic group capable of reacting with ethylene oxide can react with ethylene oxide during sterilization, absorb part of the ethylene oxide, and introduce epoxy groups, further improving the hydrophilicity of the biocompatible layer 33, thereby improving the biocompatibility of the polymer membrane 3.

In some examples, in the third-type polymer, the proportion of hydrophilic groups may be from 20% to 50%. For example, in the third-type polymer, the proportion of hydrophilic groups may be 20%, 22%, 25%, 28%, 30%, 32%, 35%, 37%, 40%, 42%, 45%, 48%, or 50%. Thus, the biocompatible layer 33 containing the third-type polymer can have good biocompatibility, thereby further improving the biocompatibility of the sensor 1. In some examples, preferably, in the third-type polymer, the proportion of hydrophilic groups may be from 30% to 50%. In some examples, preferably, in the third-type polymer, the proportion of hydrophilic groups may be not less than 30%.

In some examples, the third-type polymer may be a cross-linked water-soluble polyurethane. In this case, the water-soluble polyurethane has good biocompatibility, and thus, the formed biocompatible layer 33 can have good biocompatibility. In some examples, the third-type polymer may be polyethylene glycol block polyurethane, polymethacrylate block polyurethane, polyvinyl alcohol block polyurethane, or polypropylene alcohol block polyurethane.

In some examples, the solvent of the third solution may be an organic solvent or a mixed solvent. The third-type polymer may be dissolved in an organic solvent. Thus, the formation of the third solution and subsequently the biocompatible layer 33 can be facilitated. In some examples, the organic solvent may be a mixed solvent of any one or more of tetrahydrofuran, dichloromethane, and N,N-dimethylacetamide. Thus, the third-type polymer can be dissolved using an organic solvent. In some examples, the solvent of the third solution may include water. Thus, dissolution of the third-type polymer can be facilitated.

In some examples, the mass fraction of the third-type polymer in the third solution may be from 0.1% to 15%. For example, the mass fraction of the third-type polymer in the third solution may be 0.1%, 0.5%, 1%, 2%, 3%, 4%, 5%, 6%, 7%, 8%, 9%, 10%, 11%, 12%, 13%, 14%, or 15%. Thus, the biocompatible layer 33, having good biocompatibility, can be formed. In some examples, preferably, the mass fraction of the first-type polymer in the first solution may be from 0.1% to 5%.

In some examples, the biocompatible layer 33 may have a thickness of 1 µm to 30 µm. For example, the biocompatible layer 33 may have a thickness of 1 µm, 3 µm, 5 µm, 6 µm, 8 µm, 10 µm, 12 µm, 14 µm, 15 µm, 16 µm, 18 µm, 20 µm, 22 µm, 25 µm, 26 µm, 28 µm, or 30 µm. Thus, the biocompatibility of sensor 1 can be improved. In some examples, preferably, the biocompatible layer 33 may have a thickness of 10 µm to 20 µm.

FIG. 4 is a schematic diagram showing the polymer membrane 3 covering the implantable portion 11 according to an example of the present disclosure. In FIG. 4, the scale of the polymer membrane 3 is exaggerated for clarity, and the structure of the implantable portion 11 is simplified, but this should not be construed as limiting the present disclosure.

In some examples, the polymer membrane 3 may cover the implantable portion 11 (see FIG. 4). In some examples, the polymer membrane 3 may cover the electrodes on the implantable portion 11. For example, the polymer membrane 3 may cover the first electrode 13 and the second electrode. Thus, the first electrode 13 and the second electrode can be protected by the polymer membrane 3. As another example, the polymer membrane 3 may cover the first electrode 13, the second electrode, and the third electrode on the implantable portion 11. Thus, the electrodes can be protected by the polymer membrane 3. In some examples, the polymer membrane 3 may completely cover the implantable portion 11. Thus, the safety of sensor 1 when implanted subcutaneously in the host can be improved.

In some examples, the polymer membrane 3 may have a thickness of no greater than 100 µ m. In this case, stable adhesion of the polymer membrane 3 to the implantable portion 11 can be facilitated, and the overall space occupied by the implantable portion 11 is small, which reduces discomfort after implantation subcutaneously in the host and thereby improves wearing comfort.

In summary, in a first aspect of the present disclosure, a sensor 1 resistant to sterilizing gas can be provided.

As previously mentioned, a second aspect of the present disclosure provides an isolation layer 31 of the sensor 1. The isolation layer 31 serves to limit the permeation of sterilizing gas during gas sterilization of the sensor 1 and forms a barrier to reduce the effect of sterilizing gas on the sensing layer 14 of the sensor 1.

The isolation layer 31 in the second aspect of the present disclosure is the same as the isolation layer 31 in the sensor 1 described in the first aspect of the present disclosure, and the specific components and proportions thereof can be referred to in the description of the first aspect, and will not be described again here.

In some examples, the isolation layer 31 may be configured to reduce the effect of the sterilizing gas on the sensing layer 14 when the sensor 1 is gas sterilized. In some examples, the isolation layer 31 may cover the first electrode 13 and the sensing layer 14 of the sensor 1. Thus, the first electrode 13 and the sensing layer 14 can be protected.

In some examples, the isolation layer 31 may be made of a first solution, which may include a first-type polymer and an organic solvent. The first-type polymer may be polyurethane. In this case, the isolation layer 31 including polyurethane can limit the permeation of the sterilizing gas during the gas sterilization of the sensor 1, and forms an isolation barrier to reduce the influence of the sterilizing gas on the sensing layer 14, in particular, the influence of gaseous ethylene oxide on the active substance in the sensing layer 14, so that the sensor 1 can be sterilized by the gas sterilization method. Compared with the sensor 1 without the isolation layer 31, the decrease range of the sensitivity of the sensor 1 provided with the isolation layer 31 before and after sterilization is greatly reduced, and a higher sensitivity can be maintained after sterilization; furthermore, the isolation layer 31 containing polyurethane exhibits good biocompatibility, which can improve the safety of the sensor 1 when implanted subcutaneously in a host; in addition, the isolation layer 31 also has a permeation-limiting effect on a specific analyte (e.g., glucose), and can function as a semi-permeable membrane.

In some examples, the isolation layer 31 may also be used in other scenarios where it is desirable to limit the permeation of ethylene oxide. It may be sufficient to place the isolation layer 31 on a predetermined target. That is, the second aspect of the present disclosure may provide an isolation layer 31 capable of limiting the permeation of sterilizing gas.

In some examples, the method of preparing the isolation layer 31 may include: preparing a first solution; applying the first solution at a predetermined location; and forming the isolation layer 31 after drying.

As previously mentioned, a third aspect of the present disclosure is directed to an analyte monitoring device 100.

In some examples, the analyte monitoring device 100 may include an electronic component 2 and a sensor 1 electrically connected to the electronic component 2 (see FIG. 1). Thus, the analyte can be monitored by the sensor 1. The sensor 1 described in the third aspect of the present disclosure is identical to the sensor 1 described in the first aspect of the present disclosure, and the specific structure and details thereof can be referred to in the description of the first aspect of the present disclosure and will not be described in detail here. That is, in some examples, the analyte monitoring device 100 may include an electronic component 2 and the sensor 1 as described in the first aspect of the present disclosure.

In the analyte monitoring device 100 of the present disclosure, the sensor 1 is resistant to sterilizing gas, enabling the sensor 1 to be assembled with the electronic component 2 for gas sterilization without radiation sterilization of the sensor 1 alone.

In some examples, the analyte monitoring device 100 may be assembled as a unit at the time of shipment, and the user need not perform the additional operation of assembling the electronic component 2 with the sensor 1 at the time of use. Thus, it is possible to facilitate user operation. It should be noted that although the sensor 1 provided by the present disclosure may be of an integral design with the electronic component 2, the two may also be provided as a detachable fit, if desired. The "integral design" should not be construed as limiting the sensor 1 of the present disclosure.

In view of the foregoing, and in accordance with the present disclosure, a sterilizing gas resistant sensor 1 and its isolation layer 31 and analyte monitoring device 100 can be provided.

In order to further illustrate the present disclosure, sensor 1 and the isolation layer 31 provided by the present disclosure will be described in detail with reference to the following embodiments, and the advantageous effects achieved by the present disclosure will be fully explained with reference to the comparative examples.

It should be noted that in the embodiments and comparative examples of the present disclosure, the reagents and instruments used are commercially available products, unless otherwise specified.

First, a plurality of sensors, including a first electrode provided with a sensing layer containing glucose oxidase, is prepared for use.

### [Examples]

A first solution, a second solution, and a third solution were prepared according to Table 1-1 and Table 1-2 (a part of the embodiments had no third solution), and the second solution, the first solution, and the third solution were sequentially coated on the prepared sensors to obtain sensors having polymer membranes of Example 1 to Example 11. The PEO in Table 1-1 and Table 1-2 belonged to the hydrophilic group and soft segment, and PEGDGE referred to polyethylene glycol diglycidyl ether; PEO-containing polyurethane was taken as an example for the experiment.

**Table 1-1**

| | | First solution (isolation layer) | | Second solution (protective layer) | | Third solution (biocompatible layer) | | Membra ne layer thickness |
|---|---|---|---|---|---|---|---|---|
| | | Components | Concent ration (mass fraction) | Components | Concent ration (mg/mL ) | Compo nents | Concentratio n (mass fraction) | |
| Exam ple 1 | Solute | Polyether-based polyurethane, containing 10% PEO | 3% | (Poly-4-vinylpyridine -g- | 100 | \ | \ | Isolation layer 15 µm |
| | | | | polyethylene glycol)-co-polystyrene | | | | Protective layer 10 µm |
| | | | | PEGDGE | 15 | | | |
| | Solvent | Tetrahydrofur an | \ | 80% ethanol in water | \ | \ | \ | |
| Exam ple 2 | Solute | Polyether-based polyurethane, containing 10% PEO | 3% | (Poly-4-vinylpyridine -g- | 100 | \ | \ | Isolation layer 25 µm |
| | | | | polyethylene glycol)-co-polystyrene | | | | Protective layer 10 µm |
| | | | | PEGDGE | 15 | | | |
| | Solvent | Tetrahydrofur an | \ | 80% ethanol in water | \ | \ | \ | |
| Example 3 | Solute | Polyether-based polyurethane, containing 10% PEO | 3% | (Poly-4-vinylpyridine-g-polyethylene glycol)-co-polystyrene | 100 | \ | \ | Isolation layer 35 µm |
| | | | | | | | | Protectiv e layer 10 µm |
| | | | | PEGDGE | 15 | | | |
| | Solvent | Tetrahydrofur an | \ | 80% ethanol in water | \ | \ | \ | |
| Exam ple 4 | Solute | Polyether-based polyurethane, containing 10% PEO | 3% | (Poly-4-vinylpyridine -g-polyethylene glycol)-co-polystyrene | 100 | \ | \ | Isolation layer 40 µm |
| | | | | | | | | Protectiv e layer 10 µm |
| | | | | PEGDGE | 15 | | | |
| | Solvent | Tetrahydrofur an | \ | 80% ethanol in water | \ | \ | \ | |
| Exam ple 5 | Solute | Polyether-based polyurethane, containing 10% PEO | 3% | (Poly-4-vinylpyridine -g-polyethylene glycol)-co-polystyrene | 100 | \ | \ | Isolation layer 15 µm |
| | | | | | | | | Protectiv e layer 10 µm |
| | | | | PEGDGE | 15 | | | |
| | Solvent | Tetrahydrofur an | \ | 80% ethanol in water | \ | \ | \ | |
| Exam ple 6 | Solute | Polyether-based polyurethane, containing 10% PEO | 3% | (Poly-4-vinylpyridine -g-polyethylene glycol)-co-polystyrene | 100 | \ | \ | Isolation layer 15 µm |
| | | | | | | | | Protectiv e layer 10 µm |
| | | | | PEGDGE | 15 | | | |
| | Solvent | Tetrahydrofur an | \ | 80% ethanol in water | \ | \ | \ | |

**Table 1-2**

| | | First solution (isolation layer) | | Second solution (protective layer) | | Third solution (biocompatible layer) | | Membrane layer thickness |
|---|---|---|---|---|---|---|---|---|
| | | Components | Concentr ation (mass fraction) | Components | Concen tration (mg/m L) | Components | Concentration (mass fraction) | |
| Example 7 | Solute | Polyester-based polyurethane, containing 10% PEO | 3% | (Poly-4-vinylpyridin e-g-polyethylene glycol)-co-polystyrene | 100 | \ | \ | Isolation layer 15 µm |
| | | | | | | | | Protective layer 10 µm |
| | | | | PEGDGE | 15 | \ | \ | |
| | Solvent | Tetrahydrof uran | \ | 80% ethanol in water | \ | \ | \ | |
| Example 8 | Solute | Polycarbon ate-based polyurethane, containing 10% PEO | 3% | (Poly-4-vinylpyridin e-g-polyethylene glycol)-co-polystyrene | 100 | \ | \ | Isolation layer 15 µm |
| | | | | | | | | Protective layer 10 µm |
| | | | | PEGDGE | 15 | \ | \ | |
| | Solvent | Tetrahydrof uran | \ | 80% ethanol in water | \ | \ | \ | |
| Example 9 | Solute | Polyether-based polyurethan e, containing 10% PEO | 3% | (Poly-4-vinylpyridin e-g-polyethylene glycol)-co-polystyrene | 100 | Polyether-based polyurethan e, containing 30% PEO | 3% | Isolation layer 15 µm |
| | | | | | | | | Protective layer 10 µm |
| | | | | PEGDGE | 15 | | | Biocompat ible layer |
| | Solvent | Tetrahydrof uran | \ | 80% ethanol in water | \ | Tetrahydrof uran | \ | 15 µm |
| Example 10 | Solute | Polyether-based polyurethan e, containing 10% PEO | 3% | (Poly-4-vinylpyridin e-g-polyethylene glycol)-co-polystyrene | 100 | Polyether-based polyurethan e, containing 30% carboxybeta ine | 3% | Isolation layer 15 µm |
| | | | | | | | | Protective layer 10 µm |
| | | | | PEGDGE | 15 | | | Biocompat ible layer 15 µm |
| | Solvent | Tetrahydrof uran | \ | 80% ethanol in water | \ | Tetrahydrof uran | \ | |
| Exam ple 11 | Solute | Polyether-based polyurethan e, containing 10% PEO | 3% | (Poly-4-vinylpyridin e-g-polyethylene glycol)-co-polystyrene | 100 | Polyether-based polyurethan e, containing 30% sulfobetaine | 3% | Isolation layer 15 µm |
| | | | | | | | | Protective layer 10 µm |
| | | | | PEGDGE | 15 | | | Biocompat ible layer 15 µm |
| | Solvent | Tetrahydrof uran | \ | 80% ethanol in water | \ | Tetrahydrof uran | \ | |

### [Comparative Example 1]

The commercially available continuous glucose monitoring system (Shenzhen Silicon-based Sensing Technology Co., Ltd., model: GS1) was selected as Comparative Example 1.

### [Comparative Example 2]

The sensor of Comparative Example 2 was prepared according to the same procedure as Example 1, except that no protective layer was provided.

### [Comparative Example 3]

The sensor of Comparative Example 3 was prepared according to the same procedure as Example 4, except that the isolation layer had a thickness of 50 µm.

### [Comparative Example 4]

The sensor of Comparative Example 4 was prepared according to the same procedure as Example 1, except that the polyether-based polyurethane in the first solution was 50% PEO.

The performance of the sensor was tested:

### (1) Cytotoxicity test (biocompatibility test)

The sensors of Example 1 and Example 9 were taken as examples, cytotoxicity tests were performed, specifically: a control group and an experimental group were designed, wherein the control group included a blank group (added with blank control cell culture medium), a negative control group (added with high-density polyethylene extract solution (non-toxic substance)), and a positive control group (added with latex glove extract solution (toxic substance)); the experimental group included the first solution of Example 1 and the third solution of Example 9 at different concentrations, seeing Table 2 for details; cells were inoculated into the experimental group and the control group, incubated, and then cell viability was measured; the cytotoxicity test results were shown in Table 2.

**Table 2**

| | Group | Average absorbance | Standard deviation | Cell relative viability |
|---|---|---|---|---|
| Control group | Blank group | 1.1896 | 0.0401 | 100.00% |
| | Negative control group | 1.1246 | 0.0228 | 94.54% |
| | Positive control group | 0.0077 | 0.0034 | 0.65% |
| Example 1 | 1-1 (100% extract solution) | 1.1108 | 0.0729 | 93.38% |
| | 1-2 (50% extract solution) | 1.1293 | 0.0299 | 94.93% |
| | 1-3 (25% extract solution) | 1.1509 | 0.0435 | 96.75% |
| | 1-4 (12.5% extract solution) | 1.2187 | 0.0553 | 102.45% |
| Example 9 | 9-1 (100% extract solution) | 1.1361 | 0.0451 | 95.50% |
| | 9-2 (50% extract solution) | 1.1546 | 0.0352 | 97.06% |
| | 9-3 (25% extract solution) | 1.1762 | 0.0518 | 98.87% |
| | 9-4 (12.5% extract solution) | 1.2072 | 0.0397 | 104.57% |

### (2) Sensitivity test

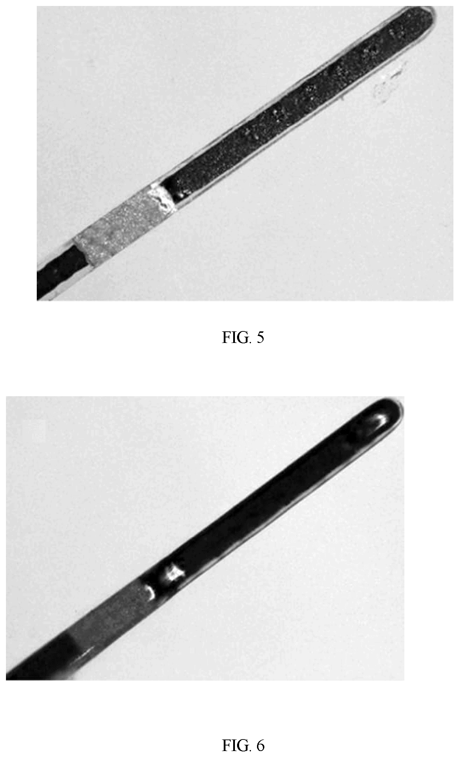

The sensors of each embodiment and each comparative example were sterilized using ethylene oxide under the following sterilization conditions: temperature: 52±3°C, humidity: 30-85%, action time: 360 min (minutes), concentration: 600±40 mg/L. In the drawings, FIG. 5 is a diagram showing the appearance of the sensor of Example 1 of the present disclosure after sterilization, and FIG. 6 is a diagram showing the appearance of the sensor of Comparative Example 1 of the present disclosure after sterilization.

Respectively test the sensitivity of each sensor before and after sterilization, specifically: the sensor was exposed to standard phosphate buffered saline (PBS buffer) with different concentrations of glucose at 37°C±1°C, the response currents at different glucose concentrations were measured, and the response currents were fitted to obtain a standard curve, so that the sensitivity of each sensor could be calculated based on the fitted standard curve. Where the glucose concentrations were respectively the following values: 2.2 mM (millimole per liter), 5 mM, 10 mM, 15 mM, 20 mM, 25 mM. The measured sensitivities are shown in Table 3. In Table 3, descending range = (sensitivity before sterilization - sensitivity after sterilization) / sensitivity before sterilization × 100%; the greater the descending range (also referred to as attenuation range), the greater the influence by ethylene oxide, and the weaker the resistance of the sensor to ethylene oxide; Conversely, the smaller the descending range, the stronger the resistance of the sensor to ethylene oxide.

**Table 3**

| | Sensitivity before sterilization (nA/mM) | Sensitivity after sterilization (nA/mM) | Reduction range |
|---|---|---|---|
| Example 1 | 1.52 | 1.32 | 13.2% |
| Example 2 | 1.23 | 1.16 | 5.7% |
| Example 3 | 0.85 | 0.82 | 3.5% |
| Example 4 | 0.65 | 0.63 | 3.1% |
| Example 5 | 1.46 | 1.37 | 6.2% |
| Example 6 | 1.60 | 1.42 | 11.3% |
| Example 7 | 1.37 | 1.19 | 13.1% |
| Example 8 | 1.25 | 1.12 | 10.4% |
| Example 9 | 1.31 | 1.18 | 9.9% |
| Example 10 | 1.35 | 1.23 | 8.9% |
| Example 11 | 1.40 | 1.22 | 12.9% |
| Comparative Example 1 | 1.64 | 0.49 | 70.1% |
| Comparative Example 2 | / | / | / |
| Comparative Example 3 | / | / | / |
| Comparative Example 4 | 2.42 | 0.21 | 91.3% |

### Analysis of results

As can be seen from FIG. 5 and FIG. 6, the appearance of the sensor of Example 1 did not significantly change after sterilization, whereas the sensor of Comparative Example 1 significantly turned green (it should be noted that, in order to comply with the provisions of *Guidelines For Patent Examination*, the drawings were converted to black and white in the present disclosure), indicating that the sensor of Comparative Example 1 without an isolation layer was eroded after sterilization.

As can be seen from Table 3, the decrease in sensitivity of the sensors of each embodiment before and after sterilization was small, indicating that the sensors of each embodiment were resistant to ethylene oxide. However, the sensitivity of the sensors of the respective comparative examples decreased significantly after sterilization, and some could not detect sensitivity. The following is a specific analysis:

In Comparative Example 1, the sensor of Comparative Example 1 could not be gas sterilized using ethylene oxide because the commercially available product did not have such an isolation layer as in the present disclosure, and the sensitivity of the sensor was greatly reduced after sterilization. In Comparative Example 2, since no protective layer was provided, the electrode was corroded after coating the first solution containing an organic solvent, resulting in undetectable sensitivity, indicating that the electrode can be effectively protected by the protective layer of the present disclosure. In Comparative Example 3, since the isolation layer had a thickness of 50 µ m, an excessive increase in membrane thickness resulted in excessive diffusion resistance of the isolation layer to glucose, and effective sensitivity could not be detected. In Comparative Example 4, the content of PEO in the isolation layer was 50%, namely, the content of soft segment and hydrophilic group was 50%; the overall hydrophilicity of the isolation layer was relatively high, and the diffusion resistance to glucose was relatively small, so that the sensor had relatively high sensitivity before sterilization, but also resulted in poor isolation effect of the isolation layer on ethylene oxide, and the sensitivity of the sensor greatly decreased after sterilization.

By comparing Example 1 with Examples 2-3, it can be seen that an increase in membrane thickness leads to a decrease in sensitivity, but also reduces the degree of sensitivity decay after sterilization, indicating that an increase in the membrane thickness of the isolation layer can reduce the damage to the electrode caused by ethylene oxide sterilization.

By comparing Example 1 with Example 4, it can be seen that since the concentration and thickness of the polyether-based polyurethane in Example 4 are both greater than those in Example 1, the degree of sensitivity decay after sterilization is reduced, but the overall sensitivity of Example 4 is lower than that of Example 1.

By comparing Example 1 with Examples 5-6, it can be seen that Examples 5-6 use different concentrations of polyether-based polyurethane to prepare the isolation layer, respectively. Due to the different solution concentrations, there are slight differences in sensitivity before sterilization and in the degree of sensitivity decay after sterilization, but no significant difference.

By comparing Example 1 with Examples 7-8, it can be seen that Examples 7-8 use different types of polyurethane, respectively. Since these two types of polyurethane are more hydrophobic than polyether-based polyurethane, the diffusion resistance of the isolation layer to glucose is greater, and the sensitivity is reduced, but the attenuation amplitude of sensitivity after sterilization is also reduced accordingly, indicating that different types of polyurethane can all achieve an isolation effect.

By comparing Example 1 with Examples 9-11, it can be seen that Examples 9-11 are sensors with biocompatible layers of different components, respectively. Due to the increased overall membrane thickness of the polymer membrane, the sensitivity of the sensors of Examples 9-11 is decreased compared with that of Example 1, but the attenuation amplitude of sensitivity after sterilization is also reduced accordingly, providing stronger protective effect; and the third solution of Example 9 showed stronger biocompatibility in the cytotoxicity test than the first solution of Example 1, indicating that adding the biocompatible layer helps improve the biocompatibility of the sensor.

In summary, the sensors of the various embodiments are highly resistant to ethylene oxide (i.e., sterilizing gas). In contrast, the sensors obtained in the comparative examples failed to achieve the performance and effects of the sensors obtained in the above-mentioned embodiments.

Although the present disclosure has been specifically described above with reference to the accompanying drawings and examples, it should be understood that the above description does not limit the present disclosure in any form. It will be apparent to those skilled in the art that various modifications and variations can be made in the present disclosure without departing from the spirit or scope of the present disclosure, which fall within the scope of the present disclosure.

## Claims

1. A biosensor for monitoring an analyte, wherein,
the biosensor comprises a first electrode provided with a sensing layer, and a polymer membrane,
the polymer membrane covers at least the sensing layer and the first electrode, the polymer membrane comprises a protective layer and an isolation layer, the protective layer is closer to the first electrode than the isolation layer,
the isolation layer is configured to reduce influence of a sterilizing gas on the sensing layer when the biosensor is subjected to gas sterilization, and the isolation layer is made of a first solution, the first solution comprises a first-type polymer and an organic solvent, and the first-type polymer is polyurethane.

2. The biosensor according to claim 1, wherein,
the mass fraction of the first-type polymer in the first solution is from 0.1% to 15%.

3. The biosensor according to claim 1 or 2, wherein,
the first-type polymer has a soft segment, and in the first-type polymer, the proportion of the soft segment is 5% to 50%.

4. The biosensor according to claim 1 or 2, wherein,
the first-type polymer has a hydrophilic group, and in the first-type polymer, the proportion of the hydrophilic group is 1% to 30%.

5. The biosensor according to claim 1 or 2, wherein,
the organic solvent is a mixed solvent of any one or more of tetrahydrofuran, dichloromethane, and N,N-dimethylacetamide.

6. The biosensor according to claim 1 or 2, wherein,
the isolation layer has a thickness of 1 µm to 40 µm.

7. The biosensor according to claim 1, wherein,
the protective layer comprises a second-type polymer, the second-type polymer is a zwitterionic polymer or a copolymer formed from neutral monomers.

8. The biosensor according to claim 1 or 7, wherein,
the protective layer has a thickness of 1 µm to 20 µm.

9. The biosensor according to claim 1, wherein,
the polymer membrane further comprises a biocompatible layer, the isolation layer is located between the biocompatible layer and the protective layer, the biocompatible layer comprises a third-type polymer, the third-type polymer is a hydrophilic group-containing polyurethane or a cross-linked water-soluble polyurethane.

10. The biosensor according to claim 9, wherein,
when the third-type polymer is the hydrophilic group-containing polyurethane, in the third-type polymer, the proportion of the hydrophilic group is from 20% to 50%.

11. The biosensor according to claim 9 or 10, wherein,
the biocompatible layer has a thickness of 1 µm to 30 µm.

12. The biosensor according to claim 1, wherein,
the sensing layer comprises one or more of glucose oxidase, lactate oxidase, and hydroxybutyrate dehydrogenase.

13. The biosensor according to claim 1, wherein,
the biosensor further comprises a second electrode different from the first electrode, and the polymer membrane also covers the second electrode.

14. An isolation layer of a biosensor, the biosensor comprising a first electrode provided with a sensing layer, wherein,
the isolation layer covers at least the sensing layer and the first electrode, the isolation layer is configured to reduce influence of a sterilizing gas on the sensing layer when the biosensor is subjected to gas sterilization, the isolation layer is made of a first solution, the first solution comprises a first-type polymer and an organic solvent, and the first-type polymer is polyurethane.

15. An analyte monitoring device, comprising an electronic component and the biosensor according to any one of claims 1 to 13, the electronic component being electrically connected to the biosensor.
